# EUROPEAN PATENT SPECIFICATION

(11) **EP 1 237 606 B1**
(45) Date of publication and mention of the grant of the patent: **17.08.2005**
(21) Application number: 00983609.9
(22) Date of filing: 29.11.2000
(51) Int. Cl.: A61M 15/00, B05B 7/14, B05B 11/06

(54) **ACTIVE WALLS**
AKTIVE WANDUNGEN
PAROIS ACTIVES

(30) Priority: 08.12.1999 SE 9904484
(43) Date of publication of application: 11.09.2002
(73) Proprietor: Microdrug AG, 6052 Hergiswil NM (CH)
(72) Inventor: NILSSON, Lars-Gunnar, S-731 30 Köping (SE); NILSSON, Thomas, S-647 32 Mariefred (SE)
(74) Representative: Mrazek, Werner
(86) International application number: PCT/SE2000/002364
(87) International publication number: WO 2001/041852

(56) References cited:
- WO-A1-94/17679
- WO-A1-98/36651
- WO-A1-99/25484

## Description

### TECHNICAL FIELD

The present invention relates to a method and a device for minimizing the amount of a fine powder sticking to the walls of a distribution member carrying a mix of air and the powder.

### BACKGROUND

Within some technical areas there is a problem in that some part of a fine powder mixed with a gas and subsequently transported by this gas, e.g. air, will stick to the walls of a distribution member when it passes through this distribution member. Instruments for measuring for instance the amount of powder mixed with a transporting gas as well as providing a measure of the grain size distribution often faces the problem that the different sizes powder grains to be measured will easily stick to the walls of a measurement channel. This implies that cleaning must be regularly performed when measuring, besides the accuracy of the measurement will be very dependent of how often measures for cleaning will be performed.

Another area facing a similar problem, for instance, is the administering of medical compositions by means of inhalers. Administering of medical powders today can be performed in numerous ways. Within health care more and more is focussed on the possibility of dosing medical drugs as a powder directly to the lungs of a patient by means of an inhaler to thereby obtain an effective, quick and patient-friendly administering of such substances.

For the medical powders, being administered by means of an inhaler, to land in the lungs, the powder should preferably have a grain size of 0.5 to 10 µm. A larger grain size will easily be sticking just in the mouth and throat, and a smaller grain size may accompany the expiration air out again.

Small grain size powder also has a strong tendency of agglomerating, i.e. to get conglomerated. In the inhalers, which are used today, a large extent of the active substance is in the form of agglomerates when it is dosed and much powder therefore will stick in the upper respiratory tract. Different ways to de-agglomerate the powder have been developed and in most cases the inhalation air is utilized for decomposing the agglomerates.

It is also common to use carriers having a larger grain size onto which the fine powder is distributed. Upon inspiration the large grains will then stick in the oral cavity while the small grains are set free and proceed to the lungs. Certain manufacturers also use electrically driven propellers, piezo-vibrators and/or mechanical vibration to decompose the agglomerates. Thus, achieving a very large portion of individual particles in the inspiratory air is a very important factor for obtaining a high degree of effectiveness upon inhalation. Yet another problem is that the medical composition powder also will stick to the walls of an inhaler distribution member normally connected to a suitable mouthpiece for the administration by means of an inspiration.

Therefore there is a demand of a solution which will to a large extent prevent particularly fine powder or its agglomerates from sticking to a distribution member, for instance, in a measuring instrument or an inhaler, which otherwise may be more or less clogged due to powder sticking to its walls.

### SUMMARY

A powder distribution device for transporting and mixing of a fine powder with a gas is disclosed. The device presents a distribution member forming a connection between a source of fine powder and a discharge opening. The distribution member has a first inlet portion and an outlet portion for a stream of a first gas mixed with the fine powder. The main body of the distribution member constitutes a porous body portion being surrounded by a second gas. If a pressure gradient is created between the second gas and the first gas, i.e. the first gas being at a slightly lower pressure, the second gas will leak trough the porous body and thereby preventing powder, in the mix of the first gas and fine powder, from sticking or clogging within the distribution member, which thereby obtains an active non-sticking wall relative to of the fine powder.

A method according the present invention is set forth by the independent claim 1 and the dependent claim 2. A device according to the invention is set fort by the independent claim 3, and further embodiments of the device are set forth by the dependent claims 4 to 10.

### BRIEF DESCRIPTION OF THE DRAWINGS

The invention, together with further objects and advantages thereof, may best be understood by making reference to the following description taken together with the accompanying drawings, in which:
- FIG. 1: illustrates a distribution member according to the present invention for connection to a source of fine powder, and
- FIG. 2: illustrates a further embodiment of a distribution member according to the present invention to regulate the amount of the second gas to obtain a desired pressure gradient.

### DETAILED DESCRIPTION

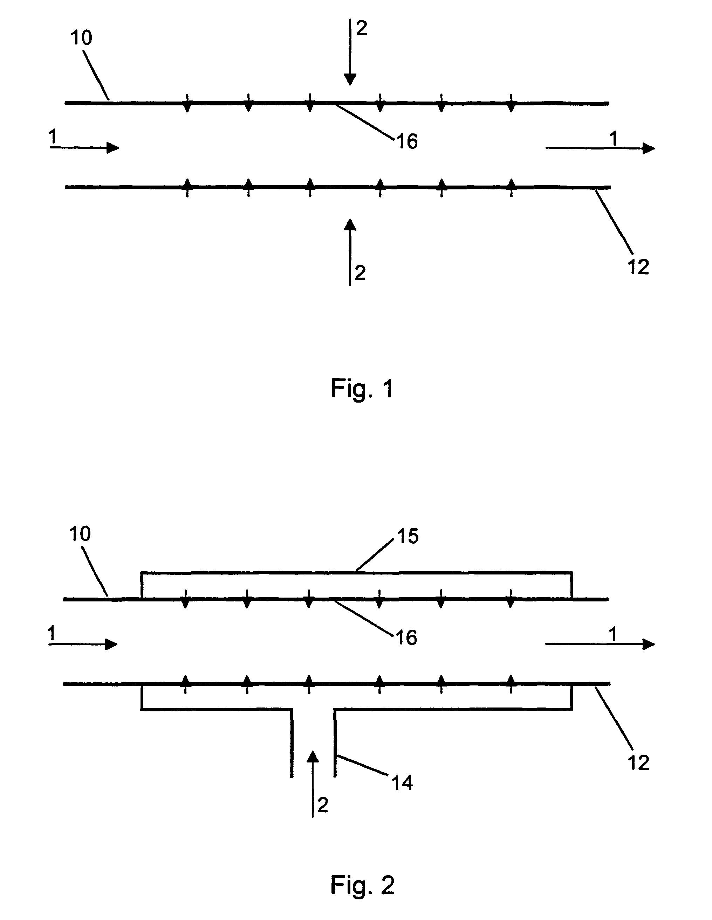

In Figure 1 is presented an embodiment of a distribution member illustrating the present invention. Such a distribution member may be used, for instance, in a measuring instrument or may even be utilized in an inhaler device. In a measurement instrument the distribution member will be inserted between a source delivering a powder to be mixed with a gas transporting the powder for forming a measurement set-up. An instrument utilizing the distribution member could typically be a measurement set-up for measuring the size of the powder particles from the powder source as well as the distribution of particle sizes when transported by a gas flow. A typical such measurement for instance may utilize a laser beam, which will be scattered by the small grains of powder transported by the gas. In an inhaler the distribution member may be inserted between a source of powder and a mouthpiece used for the inspiration of powder.

An input side 10 of the distribution member is connected to a suitable powder source. Similarly an output side 12 of the distribution member is connected to an exhaust, which in the case of an inhaler would be a suitable mouthpiece for the inhalation. The main distribution member in an illustrative embodiment consists of, for instance, a tube having a porous body 16. A first gas 1 passing through the distribution member body contains a mixture of gas and powder, for instance a medical composition to be administered by means of an inhaler. The distribution member according to a first embodiment demonstrated in Figure 1 is surrounded by a second gas 2. A pressure difference between the first gas 1 and the second gas 2 is normally obtained by creating an under-pressure in the first gas 1, for instance, by a suction of the first gas 1 from the outlet portion 12. When the second gas 2 is at a higher pressure compared to the first gas 1 the second gas 2 will leak trough the porous body portion 16 then preventing powder from sticking or clogging at the inner of the distribution member body.

In a second illustrative embodiment, for obtaining a desired pressure gradient, a second gas 2 is fed via a second inlet 14 in a second wall 15 into a space between the porous body 16 and the second wall 15. By regulating the amount of the second gas a desired pressure gradient will be obtained. The gas-flow passing through the distribution member will contain a mixture of the first gas 1 and a powder. This gas-flow then still presenting a lower pressure compared to the second gas 2 implies that the second gas 2 will leak through the porous body 16 and prevent powder from sticking to the inner wall of the porous body 16. A typical gas to be used in a measurement set-up will be air or in some set-ups dry air, but any other type of available gas may be used, for instance, even an inert gas may be used. A typical material for the distribution member body 16 is sintered porous plastic, but also porous sintered metal may be used. Preferably the material is conductive or electrostatic dissipative and connected to a device ground to prevent electrostatic charging. By balancing the pressure at the second gas inlet 14 against the pressure of the transportation gas 1, the second gas 2 when leaking through the porous body will also assist in further distributing the powder within the first gas 1. This is also valid in an inhaler device utilizing the present distribution member for improving the efficiency of the inhaler in which the pressure gradient is created by the suction of the first gas 1, in this case just ordinary air, when inhaling a medical composition powder mixed with air. In a further embodiment of an inhaler the entire portion of the inhaler case in contact with the powder to be administered may be formed of a porous material preferably enclosed in an outer shell. This outer shell then forming a space between an outer wall 15 and the inner porous body for obtaining a pressure gradient towards the interior of the device thereby, by means of the porous inner body 16, preventing powder grains from sticking to the inner body of the inhaler device.

It will be understood by those skilled in the art that various modifications and changes may be made to the present invention without departure from the scope thereof, which is defined by the appended claims.

## Claims

1. A method for minimizing the amount of a powder sticking inside a body of a distribution device carrying a gas mixed with the powder, **characterized by** the steps of
forming a distribution member body from a material presenting a porous characteristic;
creating a pressure gradient such that a lower pressure is obtained in an inner portion of the distribution member body (16), whereby a surrounding gas (2) will leak through the body of the distribution member thereby preventing powder particles from sticking to the inside of the distribution member.

2. The method according to claim 1, **characterized by** the further step of
using for the distribution member body a sintered porous plastic or a porous sintered metallic material.

3. A powder distribution device for transporting and mixing of powder with a gas **characterized in**
a distribution member body forming a connection between a source of fine powder and a discharge opening, whereby the distribution member body comprises a porous body (16) having a first inlet portion (10) and an outlet portion (12) for a stream of a first gas (1) mixed with the fine powder, the distribution member body being surrounded by a second gas (2), which when creating a pressure gradient between outer and inner regions of the distribution member body the surrounding second gas (2) will leak through a porous body (16) of the distribution member body, thereby preventing powder particles from sticking to the inside of the distribution member.

4. The device according to claim 3, **characterized in that** the porous body (16) of the powder distribution device is made from sintered porous plastic or a porous sintered metallic material.

5. The device according to claim 3, **characterized in that** the first gas (1) and the second gas (2) both constitute air.

6. The device according to claim 3, **characterized in that** a pressure difference between the first gas (1) and the second gas (2) is obtained by creating an under-pressure in the first gas (1) by a suction of the first gas (1) from the outlet portion (12).

7. The device according to claim 3, **characterized in that** the powder distribution device further constitutes an outer wall portion (15) encircling an inner porous body (16) and the outer wall portion (15) further being provided with a second inlet portion (14) for an inlet of the second gas (2) at a higher pressure compared to the first gas (1) to have the second gas (2) leak trough the inner porous body portion (16) thereby preventing powder from sticking or clogging at inner walls of the distribution member.

8. The device according to claim 3, **characterized in that** the distribution member at its first inlet portion (10) also constitutes a source of powder to thereby form an inhaler arrangement connected to a mouthpiece for inhaling the powder, whereby due to active inner walls of the device powder will not stick to inner walls of the inhaler.

9. The device according to claim 7, **characterized in that** a pressure difference between the first gas (1) and the second gas (2) is obtained by adjusting the pressure of the second gas (2) fed at the second inlet portion (14).

10. The device according to claim 4, **characterized in that** the porous body (16) is conductive or electrostatic dissipative and connected to a device ground to prevent electrostatic charging.

## Patentansprüche

1. Verfahren zum Minimieren der Menge eines Pulvers, das innerhalb eines Körpers einer Verteilungsvorrichtung, welche ein mit dem Pulver gemischtes Gas transportiert, anhaftet, **gekennzeichnet durch** die Schritte:
Bilden eines Verteilungselementkörpers aus einem Material, das eine poröse Eigenschaft aufweist;
Erzeugen eines Druckgefälles, so dass in einem Innenbereich des Verteilungselementkörpers (16) ein niedrigerer Druck erhalten wird, wobei ein Umgebungsgas (2) **durch** den Körper des Verteilungselements strömt, wodurch verhindert wird, dass Pulverteilchen an der Innenseite des Verteilungselements anhaften.

2. Verfahren nach Anspruch 1, **gekennzeichnet durch** den weiteren Schritt der Verwendung eines gesinterten porösen Kunststoffes oder eines porösen gesinterten Metallmaterials für den Verteilungselementkörper.

3. Pulververteilungsvorrichtung zum Transportieren und Mischen von Pulver mit einem Gas, **gekennzeichnet durch**
einen Verteilungselementkörper, der eine Verbindung zwischen einer Quelle von feinem Pulver und einer Ablassöffnung bildet, wobei der Verteilungselementkörper einen porösen Körper (16) mit einem ersten Einlassbereich (10) und einem Auslassbereich (12) für einen mit dem feinen Pulver gemischten Strom eines ersten Gases (1) umfasst, wobei der Verteilungselementkörper von einem zweiten Gas (2) umgeben ist, das **durch** einen porösen Körper (16) des Verteilungselementkörpers strömt, wenn ein Druckgefälle zwischen Außen- und Innenbereichen des Verteilungselementkörpers erzeugt wird, wodurch verhindert wird, dass Pulverteilchen an der Innenseite des Verteilungselements anhaften.

4. Vorrichtung nach Anspruch 3, **dadurch gekennzeichnet, dass** der poröse Körper (16) der Pulververteilungsvorrichtung aus einem gesinterten porösen Kunststoff oder einem porösen gesinterten Metallmaterial hergestellt ist.

5. Vorrichtung nach Anspruch 3, **dadurch gekennzeichnet, dass** das erste Gas (1) und das zweite Gas (2) beide Luft sind.

6. Vorrichtung nach Anspruch 3, **dadurch gekennzeichnet, dass** ein Druckunterschied zwischen dem ersten Gas (1) und dem zweiten Gas (2) durch Erzeugen eines Unterdrucks im ersten Gas (1) durch ein Ansaugen des ersten Gases (1) aus dem Auslassbereich (12) erhalten wird.

7. Vorrichtung nach Anspruch 3, **dadurch gekennzeichnet, dass** die Pulververteilungsvorrichtung ferner einen Außenwandbereich (15) aufweist, der einen inneren porösen Körper (16) umgibt, und dass der Außenwandbereich (15) ferner mit einem zweiten Einlassbereich (14) für ein Einlassen des zweiten Gases (2) mit einem höheren Druck im Vergleich zum ersten Gas (1) ausgestattet ist, damit das zweite Gas (2) durch den inneren porösen Körperbereich (16) strömt kann, wodurch verhindert wird, dass Pulver an Innenwänden des Verteilungselements anhaftet oder diese verstopfen kann.

8. Vorrichtung nach Anspruch 3, **dadurch gekennzeichnet, dass** das Verteilungselement an seinem ersten Einlassbereich (10) auch eine Quelle des Pulvers aufweist, um somit eine Inhalationsanordnung zu bilden, die mit einem Mundstück zum Inhalieren des Pulvers verbunden ist, wobei aufgrund der aktiven Innenwände der Vorrichtung kein Pulver an den Innenwänden des Inhalators anhaftet.

9. Vorrichtung nach Anspruch 7, **dadurch gekennzeichnet, dass** ein Druckunterschied zwischen dem ersten Gas (1) und dem zweiten Gas (2) durch Anpassen des Drucks des zweiten Gases (2), das am zweiten Einlassbereich (14) zugeführt wird, erhalten wird.

10. Vorrichtung nach Anspruch 4, **dadurch gekennzeichnet, dass** der poröse Körper (16) leitend oder elektrostatisch ableitend ist und mit einer Erdung der Vorrichtung verbunden ist, um eine elektrostatische Aufladung zu verhindern.

## Revendications

1. Procédé pour minimiser la quantité de collage de poudre à l'intérieur d'un corps d'un dispositif de distribution transportant un gaz mélangé à la poudre, **caractérisé par** les étapes consistant à
former un corps d'élément de distribution à partir d'un matériau présentant une caractéristique poreuse ;
créer un gradient de pression de telle sorte qu'une pression inférieure soit obtenue dans une partie intérieure du corps d'élément de distribution (16), de sorte qu'un gaz englobant (2) fuira à travers le corps de l'élément de distribution empêchant ainsi les particules de poudre de coller à l'intérieur de l'élément de distribution.

2. Procédé selon la revendication 1, **caractérisé par** l'étape supplémentaire consistant à
utiliser pour le corps d'élément de distribution un plastique poreux fritté ou un matériau métallique fritté poreux.

3. Dispositif de distribution de poudre pour transporter et mélanger une poudre avec un gaz **caractérisé par**
un corps d'élément de distribution formant un raccordement entre une source de poudre fine et une ouverture d'évacuation, dans lequel le corps d'élément de distribution comprend un corps poreux (16) comprenant une première partie d'admission (10) et une partie de refoulement (12) pour un jet d'un premier gaz (1) mélangé à la poudre fine, le corps d'élément de distribution étant entouré d'un second gaz (2), de sorte que lors de la création d'un gradient de pression entre des régions extérieure et intérieure du corps d'élément de distribution, le second gaz englobant (2) fuira à travers un corps poreux (16) du corps d'élément de distribution, empêchant ainsi aux particules de poudre de coller à l'intérieur de l'élément de distribution.

4. Dispositif selon la revendication 3, **caractérisé en ce que** le corps poreux (16) du dispositif de distribution de poudre est composé de plastique poreux fritté ou d'un matériau métallique fritté poreux.

5. Dispositif selon la revendication 3, **caractérisé en ce que** le premier gaz (1) et le second gaz (2) constituent de l'air.

6. Dispositif selon la revendication 3, **caractérisé en ce qu'**une différence de pression entre le premier gaz (1) et le second gaz (2) est obtenue en créant une sous-pression dans le premier gaz (1) par une aspiration du premier gaz (1) à partir de la partie de refoulement (12).

7. Dispositif selon la revendication 3, **caractérisé en ce que** le dispositif de distribution de poudre constitue en outre une partie de paroi extérieure (15) entourant un corps poreux intérieur (16) et la partie de paroi extérieure (15) étant en outre pourvue d'une seconde partie d'admission (14) pour une admission du second gaz (2) à une pression supérieure par rapport au premier gaz (1) pour que le second gaz (2) fuie à travers la partie de corps poreux intérieur (16), empêchant ainsi la poudre de coller ou de s'accumuler sur les parois intérieures de l'élément de distribution.

8. Dispositif selon la revendication 3, **caractérisé en ce que** l'élément de distribution au niveau de sa première partie d'admission (10) constitue également une source de poudre pour former ainsi un dispositif d'inhalateur relié à une bouche pour inhaler la poudre, de sorte qu'en raison des parois intérieures actives du dispositif, la poudre ne collera pas sur les parois intérieures de l'inhalateur.

9. Dispositif selon la revendication 7, **caractérisé en ce qu'**une différence de pression entre le premier gaz (1) et le second gaz (2) est obtenue en ajustant la pression du second gaz (2) amené au niveau de la seconde partie d'admission (14).

10. Dispositif selon la revendication 4, **caractérisé en ce que** le corps poreux (16) est conducteur ou dissipe l'énergie électrostatique et est relié à un dispositif relié à la terre afin d'éviter une charge électrostatique.
